# EUROPEAN PATENT APPLICATION

(11) **EP 1 785 414 A1**
(43) Date of publication of application: **16.05.2007**
(21) Application number: 05024427.6
(22) Date of filing: 09.11.2005
(51) Int. Cl.: C07C 315/04, C07C 317/32

(54) **Process for the synthesis of intermediates of chloramphenicol or its analogues**

(71) Applicant: KRKA, tovarna zdravil, d.d., Novo mesto, 8501 Novo mesto (SI)
(72) Inventor: Gnidovec, Joze, 8222 Otocec (SI); Kolenc, Ivanka, 8000 Novo mesto (SI)
(74) Representative: Becker Kurig Straus

(57) **Abstract**

The present invention relates to the synthesis of antibacterial compounds such as Chloramphenicol and its analogues Thiamphenicol and Florfenicol and particularly to a new reaction for the preparation of the intermediate compound aminodiolphenylsulfone. This reaction permits the introduction of modified residues to obtain modified antibiotics with an improved stability towards the action of bacterial resistant determinants. In addition, higher purities may be also obtained due to an improved procedure requiring fewer purification steps.

## Description

The present invention relates to the synthesis of antibacterial compounds such as Chloramphenicol and its analogues and particularly to a new reaction for the preparation of the intermediate compound aminodiolphenylsulfone (ADS), required for the synthesis of Thiamphenicol and Florfenicol. This reaction allows the introduction of modified residues to obtain modified antibiotics with an improved stability towards the action of bacterial resistant determinants. In addition, higher purities may be also obtained due to an improved procedure requiring fewer purification steps.

In view of the emerging resistance of numerous bacterial strains towards antibiotic compounds, there is a continuous need in the development of bacteriostatics exhibiting new structures rendering them less vulnerable to the action of resistant bacterial strains. A possibility to provide such new structures resides in the modification of already known antibiotics either by modifying the structure of the final synthesis' product or already performing structural changes at the stage of the intermediate compounds.

Known antibiotics of interest comprise for example the compound Chloramphenicol and its analogues Thiamphenicol and Florfenicol or other related substances. Said compounds exhibit a strong antibacterial activity for Gram-positive and Gram-negative bacteria rendering them useful for controlling infections in both animals and humans. It has been established that Florfenicol manifests the best antibacterial effect. Thiamphenicol is inferior to Florfenicol and Chloramphenicol in terms of antibacterial activity in vitro, especially with respect to some Gram-positive microorganisms. Florfenicol's antibiotic effect is similar to that of Chloramphenicol and Thiamphenicol. It mainly restrains the combination of the bacterium 70S ribosome and the 50S subunit, controls the peptidyl to shift enzyme, restraining the peptide chain's extending, disturbs the bacterial protein's synthesis. Bacterial resistance to Chloramphenicol emerges due to acetylation, hydrolysis to p-Nitrophenylserinol and reduction of the nitro group, whereas Thiamphenicol and Florfenicol are not influenced by acetylation and may even inhibit bacterial acetyltransferases.

A challenge in the chemical synthesis resides in the creation of stereo centers to obtain the compounds having the desired activity. In the WO 9414764 for example a process for the asymmetric synthesis of Florfenicol, Thiamphenicol or Chloramphenicol is described. A process for isomerizing the S,S-isomer of Florfenicol to the R,S-isomer may be performed by sequentially treating with: (i) a lower alkylsulfonyl chloride and a tertiary amine base; (ii) sulfuric acid and water; (iii) an alkali metal hydroxide. In addition, a process for regioselectively opening an epoxide to form a threo-oxazoline is described.

Another synthesis of Thiamphenicol is described for example in the US-P-3,733,352. P-Methylsulfonylbenzaldehyde is converted in presence of Aminoacetic acid and an alkali metal carbonate in an alcoholic solvent to racemic β-p-Methylsulfonylphenylserine. After esterification with an alcohol in acidic environment, the respective ester is subjected to a racemization to obtain the desired D-form. Thiamphenicol is obtained after a reduction step with Sodium borohydride and Calcium chloride or Lithium aluminum hydride (LAH) and an additional reaction step with Trichloro actetaldehyde and alkali metal cyanide.

According to the US-P-3,927,054 DL-threo-beta-p-methylsulfonylphenylserines as intermediates for the synthesis of Thiamphenicol are synthesized by reacting p-Methylsulfonylbenzaldehyde with glycine copper complex in a suitable solvent under basic conditions and esterifying the resulting DL-threo- beta -p-Methylsulfonylphenylserine copper complex with an alcohol. The residual reaction steps to the final product are essentially the same as disclosed in US-P-3,733,352 as stated above. According to the US-P-2,816,915, the reduction step may be performed with Lithium aluminum hydride.

An alternative synthesis for Thiamphenicol is proposed in the EP-P-0224902 and US-A-2005/0075506. Herein, as an intermediate compound, D-(-)-threo-2-amino-1-[4-(methylsulfonyl)phenyl]-1,3-propanediol hydrochloride as an example of Aminodiolsulfone hydrochloride (ADS hydrochloride) is mentioned. ADS hydrochloride may be subjected to a racemate separation and acetylated to obtain the final product. The synthesis of ADS hydrochloride is performed by conversion of the respective Nitro-compound with Pd/C as catalyst. Another method for the synthesis of ADS hydrochloride is disclosed in US-P-4,235,892 by hydrolysis of Thiamphenicol with hydrochloric acid. Both methods have major drawbacks rendering them not attractive for industrial processes

In view of emerging resistance towards antibiotics, there is a continuous need in the development of both antibiotics with new structures and synthesis methods allowing to obtain such structures. There is also a need in the provision of new intermediates of Chloramphenicol and its analogs and new reactions/reaction schemes to provide said compounds in order to provide an improved synthesis offering a facilitated purification of intermediate compounds, higher yields and the possibility to convert residues easily and selectively to obtain new antibiotics having less susceptibility to degrading/modifying enzymes of bacterial strains with resistance determinants.

A problem of the present invention is, therefore, the provision of a new reaction, which may be implemented in the industrial synthesis of Chloramphenicol or its analogues.

The present invention solves the above-mentioned problem by the provision of a process for the synthesis of ADS acid, comprising the steps of (i) reduction of a phenylsulfone serine ester with borohydride; (ii) conversion of the reduced phenylsulfone serine ester with an acid to obtain crude ADS-acid; and (iii) purification of crude ADS-acid to obtain the purified final product.

The present inventors have surprisingly found out that aminodiolsulfone or aminodiolphenylsulfone may be isolated in the form of hydrochloride after a reduction step and may be used without conversion to the base for further reaction. This permits on the on hand omission of a reaction step in the synthesis of Chloramphenicol analogues, improved yields and purity. Another advantage resides in that the sulfone group of the phenylsulfone serine ester may comprise different residues, which are affecting on the one hand the antibacterial activity of the respective final synthesis product and which are not affect by the reductant.

According to a first embodiment of the present invention, a process for the synthesis of an ADS-acid is provided. Said process comprises the steps of (i) reducing a phenylsulfone serine ester with borohydride; (ii) converting the aminodiolphenylsulfone with an acid to obtain crude aminodiolphenylsulfone-acid; and (iii) purifying crude aminodiolphenylsulfone-acid.

The term borohydride as used herein relates to any compound comprising BH₄⁻ having a reductive capacity. The advantage of borohydride resides in an increased selectivity of the reaction site since other reductants, e.g. Lithium aluminum hydride, are often more reactive and consequently less specific by converting other residues. Additionally, the present inventors have surprisingly found that the reaction product achieved after the conversion borohydrides requires less purification steps than in case are other metal hydrides such as Lithium aluminum hydride are used. E.g. the US-P- 2,816,915 specifies the reduction of a serine ester with Lithium aluminum hydride and isolation of the reaction product via oxalate, requiring several purification steps and providing low yields.

In a first step to phenylsulfone serine ester borohydride is added to a polar solvent, preferably protic solvent. The reaction mixture is stirred and/or kept at a certain temperature for a predetermined time. Afterwards, the polar solvent is removed. The solvent may be removed according to various techniques well known to the skilled persons. Such techniques comprise e.g. distillation or extraction and are accomplished until a predetermined degree of purity is obtained, which may be determined via known techniques, e.g. High Performance Liquid Chromatography (HPLC). The term "polar" as used herein refers to the dielectricity constant of the respective solvent, in that for example the term polar solvent designates each solvent with the same or higher dielectricity constant like ethanol, such as methanol. In a second process step the crude ADS obtained is converted by the addition of an acid to the respective crude ADS-acid. Also this step may be performed at a certain temperature for a predetermined time. The crude ADS-acid is further purified to obtain the purified ADS-acid.

Purification techniques and protocols are also well known to the skilled person and may be easily adapted to changing requirements. The expression "crude" refers to any raw compound obtained via synthesis, which is not subjected to any subsequent purification step. The purification of a crude compound may be for example performed by condensing, filtering and drying, alternatively for example re-crystallization and chromatographic techniques are suitable and may be combined to yield a purified or partially purified product. Also the purification may be performed by employing different solvents, preferably organic solvents, which are known to the skilled person.

According to another embodiment the acid used may be selected from the group consisting of hydrochloric acid, acetic acid, and sulfuric acid. The advantage of the conversion of crude ADS prior purification resides in a facilitated purification protocol requiring fewer reaction steps and affording higher purities of the intermediate product. Chromatographic purities obtained were in case of ADS hydrochloride 97.94%, ADS acetate 96.79% and ADS sulfate 96.14%.

The reactant phenylsulfone serine ester may have a sterochemical configuration as required and may be prepared by already known techniques. The phenylsulfone serine ester, aminodiolphenylsulfone and aminodiolphenylsulfone-acid may comprise different residues R at the phenylsulfone group, which is located at the position C₃ of the serine. The sulfone of the phenylsulfone group is preferably located in the para position. Examples of the residue R comprise hydrogen, alkane and aryl residues, preferably R=CH₃. The ester may be any ester such as an alkyl, such as ethyl, or aryl ester. A preferred phenylsulfone serine ester is for example D-(-)-threo-p-methylsulfonylphenyl serine ethyl ester.

The borohydride in process step (i) may be any suitable borohydride. Examples for such borohydrides are sodium and potassium borohydride.

According to still another embodiment the obtained purified ADS-acid is further converted with subsequent reaction steps to Chloramphenicol analogs, such as Thiamphenicol and preferably Florfenicol, which synthesis is exemplified hereinafter. It should be clear to the skilled that the present process is also suitable for the synthesis of Chloramphenicol if instead of a phenylsulfone serine ester a protected p-nitrophenyl serine ester is used as starting compound.

Florfenicol may be obtained starting from D-(-)-threo-p-methylsulfonylphenyl serine ethyl ester by reduction with potassium borohydride, converting the reaction product with acidic acid and purification according to the present process. D-(-)-threo-2-amino-1-[4-(methylsulfonyl)phenyl]-1,3-propanediol hydrochloride obtained may be further converted with benzonitrile to provide a protection group. The Oxazoline obtained from ADS hydrochloride also exhibits a surprising high HPLC purity of 99.76%. Alternatively, the oxazoline may be prepared directly form the serine ester resulting in a HPLC purity of 96.22%. The oxazoline is further fluorinated, e.g. by means of a fluorination agent like hexafluoropropylene gas. In a subsequent reaction step the protecting group is removed by hydrolysis in acidic environment. Crude Florfenicol is obtained by acetylation with dichloroacetate, which may be purified according to standard protocols.

By employing suitable purification techniques known to the skilled person crude Chloramphenicol or it analogues may be purified to a degree rendering it suitable for treatment purposes. Chloramphenicol or its analogues, preferably Florfenicol, may be incorporated in solutions for injections by mixing either with propylene glycol, Dimethylsulfoxid, Macrogol 400 or with N-methyl-2-pyrrolidone, Propylene glycol and Polyethylene glycol in certain amounts known to the skilled person. In the process of preparing injection solution it should be assured that water is absent from manufacturing process (equipment). Alternatively, Chloramphenicol or its analogues, preferably Florfenicol, may be incorporated in medicated feed comprising Lactose Monohydrate and Silica, Colloidal Anhydrous or Polyethylene glycol 200 in certain amounts known to the skilled person.

The following examples illustrate the invention without limiting it thereto.

### Examples

### Example 1

D-(-)-threo2-amino-4-[4-(methylsulfonyl)phenyl]-1,3-propanediol hydrochloride 50 g (174.0mmol) of D-(-)-threo-p-methylsulfonylphenyl serine ethyl ester, 14 g (259.6mmol) of potassium borohydride and 200 ml of methanol were charged in a 500 ml flask. The mixture was stirred at room temperature. Stirring was continued and the temperature controlled in a range of 50 to 55°C for 2 hours. Methanol was distilled off at 30-40°C in vacuum and a yellow oil was obtained. Then 150 ml (420 mmol) of 2.8 mol/L hydrochloride -ethanol was added, heated to reflux for 30 minutes and filtered to remove insoluble mass. The filtrate was condensed at 50-60°C in vacuum to thick slurry, 25 ml of isopropanol was added to the thick slurry, stirred for 15 minutes (the mixture also was slurry), the slurry was chilled to 0-5°C for 30 minutes, the solid filtered off and washed with 20 ml of chilled isopropanol. The solid was dried at 70°C for 4 hours. 41.1 g of the titled compound were obtained (145.5 mmol); yield 83%. The amount of diol-hydrochloride was assessed using HPLC with an Agilent C18 column (250× 4.6, 5µm); detector: UV225 nm; flow rate: 0.8 ml/min; mobile phase: methanol-buffer (pH 4.5, KH₂PO₄) 10:90(v/v); injection volume: 10µl (0.3mg/ml).

The polymorphic form of the product was characterized by X-ray diffraction pattern shown below:

| No. | Angle | d | Rel. Int. |
|---|---|---|---|
| | [°2Th.] | [L] | [%] |
| 1 | 9.1175 | 9.69959 | 2.61 |
| 2 | 9.5269 | 9.28368 | 5.68 |
| 3 | 14.0025 | 6.32480 | 23.07 |
| 4 | 14.6689 | 6.03892 | 7.41 |
| 5 | 14.9962 | 5.90787 | 5.02 |
| 6 | 15.5314 | 5.70546 | 4.68 |
| 7 | 15.9014 | 5.57355 | 3.89 |
| 8 | 16.9863 | 5.21992 | 39.26 |
| 9 | 18.0017 | 4.92772 | 54.63 |
| 10 | 18.2752 | 4.85457 | 33.87 |
| 11 | 18.8437 | 4.70938 | 91.70 |
| 12 | 19.1017 | 4.64635 | 100.00 |
| 13 | 20.7748 | 4.27580 | 34.19 |
| 14 | 21.4836 | 4.13629 | 15.70 |
| 15 | 22.0231 | 4.03618 | 36.07 |
| 16 | 22.5349 | 3.94566 | 43.51 |
| 17 | 23.7598 | 3.74494 | 12.61 |
| 18 | 25.1487 | 3.54118 | 15.78 |
| 19 | 26.1143 | 3.41240 | 23.64 |
| 20 | 26.4571 | 3.36895 | 15.53 |
| 21 | 27.1956 | 3.27912 | 37.89 |
| 22 | 27.5401 | 3.23888 | 42.03 |
| 23 | 27.7768 | 3.21181 | 25.29 |
| 24 | 27.9949 | 3.18729 | 26.09 |
| 25 | 28.1986 | 3.16472 | 38.76 |
| 26 | 28.5893 | 3.12236 | 14.76 |
| 27 | 30.4938 | 2.93155 | 31.31 |

### Example 2

D-(-)-threo-2-phenyl-4-[4-(methylsulfonyl)phenyl] -2-oxazole-5-methanol
600 ml of methanol was charged in the reactor; 200g of D-(-)-threo-p-methylsulfonylphenyl serine ethyl ester was added to the reactor under stirring at room temperature and 56 g of potassium borohydride was added under stream of nitrogen gas. The mixture was cooled to 35°C and the reacting temperature was controlled in range of 50 to 55°C under stirring for 2 hours, methanol was distilled off at normal atmosphere until 80°C of vapour, then in vacuum for 10 mins. The residue was cooled to rt. and 2.8 mol/L 600 ml of hydrochloride-ethanol were added. The solution was cooled to - 5°C for 2 hours under stirring. The mass was filtered and the cake washed with 50 ml of ethanol chilled to - 5°C and D-(-)-threo-2-amino-4-[4-(methylsulfonyl)phenyl]-1,3-propanediol hydrochloride was collected.

750g of glycerol and the obtained product from the previous step were charged in the reactor. 65g of potassium carbonate was added portion by portion( 10g per portion), the mixture was heated to 80°C under stirring and 130 ml of benzonitrile were added to the mixture. The mixture was heated to 105 to 110°C under stirring for 10 hours then the mixture was cooled to 80°C, 1200 ml of water was added under stirring and the mixture was chilled to 15°C for 1 hour under stirring. The precipitate was filtered and washed with a chilled mixture of isopropanol and water. The product obtained was dried at 80°C for 10 hours. The yield obtained was around 70% (mole ratio).

### Example 3

D-(-)-threo-2-phenyl-4-(fluoromethyl)-5-[4-(methylsulfonyl)phenyl]-2-oxazoline
230 ml of methylene chloride and 17.5g of diethylamine were charged in the reactor. Then under cooling 37g of hexafluoropropylene gas was added to the reactor, warmed to -5°C and 50g of D-(-)-threo-2-phenyl-4-[4-(methylsulfonyl)phenyl] -2-oxazole-5-methanol was charged. The mixture was heated slowly to 105 - 110°C, meanwhile generating the pressure; the conditions were kept for 40 minutes. The mixture was cooled to room temperature and the solution was washed with aqueous NaOH, aqueous phase separated. Methylene chloride was distilled off and 200 ml of isopropanol was charged in the residue; isopropanol was partly distilled off, the solution was cooled and the precipitate filtered to obtain the product. The product was dried at 50°C.

### Example 4

### Flofenicol

410 ml water, 85g of D-(-)-threo-2-phenyl-4-(fluoromethyl)-5-[4-(methylsulfonyl)phenyl]-2-oxazoline and 540g of concentrated hydrochloric acid were provided. The mixture was heated to 105 -110°C under stirring and kept under reflux for 2 hours; 500 ml of toluene were added, the organic phase was separated and the aqueous phase was condensed until the volume of the residue was about 110 ml, and then 140 ml of water was added. 300 ml of methylene chloride was added, the solution was washed with aqueous NaOH. After extraction the extracted methylene chloride was distilled, until the volume of residue was 130 to 150 ml. Methanol was added in the residue and then the solvent was partly distilled off. 36 ml of triethylamine was added to the residue, and then 75 ml of methyl dichloroacetate and the solution was stirred at 30°C for 12 hours. 300 ml of water were added to the solution under stirring and then slowly 180 ml of toluene, then the reaction mixture was cooled, the precipitate was filtered and washed with toluene and water. After drying the material at 75°C for 8 hours 75 g of the product is obtained. The product was recrystallized from water: isopropanol 1:1 solvent mixture.

### Composition Example 1

### Solution for injection 300 mg/ml

| Ingredients | quantity |
|---|---|
| Florfenicol | 30.000 g |
| Propylene glycol | 15.000 g |
| Dimethyl sulfoxide | 25.000 g |
| Macrogol 400 | 48.125 g |

Macrogol was charged in a stainless steel tank. During agitation propylene glycol and dimethyl sulfoxide was added. The mixture was heated to 60 - 65 °C and agitated. Florfenicol was added and agitated until dissolved. The prepared solution was filtered (through a pre-filter and sterile 0.2 µm filter) and filled into vials. The filled vials were terminally sterilized at 121 °C for 20 minutes.

### Composition Example 2

### Solution for injection 300mg/ml

| Ingredients | Quantity |
|---|---|
| Florfenicol | 300 mg |
| N-methyl-2-pyrrolidone | 250 mg |
| Propylene glycol | 150 mg |
| Polyethylene glycol | Qs ad. 1 ml |

N-methyl pyrrolidone, propylene glycol and 90% of polyethylene glycol required, were mixed and then Florfenicol was dissolved in the mix. The volume was adjusted with remaining polyethylene glycol, the clear solution was sterilized by filtration and filled into vials.

### Composition Example 3

### 100 g premix for medicated feed

| Ingredients | Quantity |
|---|---|
| Florfenicol | 2.00 g |
| Lactose Monohydrate | 97.50 g |
| Silica, Colloidal Anhydrous | 0.50 g |

Florfenicol, a part of lactose monohydrate and silica colloidal anhydrous were sieved and mixed together in a mixer. Homogenized mixture was added to the remaining part of lactose monohydrate and homogenized in a suitable mixer.

### Composition Example 4

### Oral solution 100mg/ml

| Ingredients | Quantity |
|---|---|
| Florfenicol | 50.00 kg |
| Polyethylene glycol 200 | 500.00 ml |

Florfenicol was added to polyethylene glycol 200 at 60 -65 °C and mixed until dissolved. The solution was cooled to 30-35 °C. The solution was filtered through a pre-filter Millipore AP 25 under the pressure of nitrogen and collected in a filling tank. The solution in the filling tank was overlayed with nitrogen. The solution was filled into the bottles by introducing nitrogen.

## Claims

1. A process for synthesis of aminodiolphenylsulfone-acid, comprising the steps of
(i) reducing a phenylsulfone serine ester with borohydride;
(ii) converting the aminodiolphenylsulfone with an acid to obtain crude ADS-acid; and
(iii) purifying crude ADS-acid.

2. The process according to claim 1, wherein the acid is selected from the group consisting of hydrochloric acid, acidic acid and sulfuric acid.

3. The process according to any of the preceding claims, wherein said phenylsulfone serine ester is D-(-)-threo-p-methylsulfonylphenyl serine ethyl ester.

4. The process according to any of the preceding claims, wherein said borohydride is selected from the group consisting of sodium and potassium borohydride.

5. The process according to any of the preceding claims, wherein the reduction of the phenylsulfone serine ester in step (i) is further **characterized by** using a polar solvent.

6. The process according to any of the preceding claims, wherein the conversion with an acid to obtain crude aminodiolphenylsulfone-acid in step (ii) is performed in presence of a polar solvent.

7. The process according to claim 5, wherein the polar solvent is methanol and/or ethanol.

8. The process according to any of the preceding claims, wherein the purified crude ADS-acid obtained in step (iii) is further converted to Chloramphenicol or its analogs.

9. The process according to claim 8, wherein the Chloramphenicol analog is selected from the group consisting of Thiamphenicol and Florfenicol.
